# EUROPÄISCHE PATENTSCHRIFT

(11) **EP 0 896 814 B1**
(45) Veröffentlichungstag und Bekanntmachung des Hinweises auf die Patenterteilung: **13.11.2002**
(21) Anmeldenummer: 98114973.5
(22) Anmeldetag: 10.08.1998
(51) Int. Cl.: A61F 13/15

(54) **Windel- oder Inkontinenzhöschen zum einmaligen Gebrauch**
Disposable diaper or panty diaper
Couche ou couche-culotte à usage unique

(30) Priorität: 14.08.1997 DE 19735304; 14.08.1997 DE 19735303
(43) Veröffentlichungstag der Anmeldung: 17.02.1999
(73) Patentinhaber: Paul Hartmann Aktiengesellschaft, 89522 Heidenheim (DE)
(72) Erfinder: Gause, Enno, Dr. rer. nat., 89522 Heidenheim (DE); Benning, Heiner, Dipl.-Ing., 73257 Köngen (DE); Hermann, Klaus, 89537 Giengen (DE)
(74) Vertreter: Dreiss, Fuhlendorf, Steimle & Becker

(56) Entgegenhaltungen:
- EP-A- 0 689 816
- US-A- 5 213 645
- US-A- 5 607 416

## Beschreibung

Die Erfindung betrifft ein Verfahren zum Herstellen eines Windel- oder Inkontinenzhöschens zum einmaligen Gebrauch, mit einem Hauptteil, bestehend aus einem Vorderbereich, einem Rückbereich und einem in Längsrichtung dazwischen liegenden im Schrittbereich des Benutzers des Höschens zu liegen kommenden Mittelbereich, wobei der Hauptteil eine die körperabgewandte Außenseite bildende, zumindest abschnittsweise flüssigkeitsdichte Schicht, einen Saugkörper und eine die körperzugewandte Innenseite bildende, zumindest im Bereich des darunter angeordneten Saugkörpers flüssigkeitsdurchlässige Schicht aufweist, und mit elastischen Materialabschnitten, welche den Vorderbereich und den Rückbereich zur Bildung der Höschenform miteinander verbinden und sich in senkrecht zur Längsrichtung verlaufender Querrichtung über seitliche Längsränder des Hauptteils hinaus erstrecken, wobei die elastischen Materialabschnitte einen gürtelförmigen in Umfangsrichtung geschlossenen Hüftabschnitt bilden, der am Rückbereich und am Vorderbereich des Hauptteils unlösbar befestigt ist.

Ein derartiges Windelhöschen ist aus der US Patentschrift 5,607,416 bekannt; nach einer Ausführungsform ist der Hüftgurt in Umfangsrichtung nämlich durchgehend geschlossen ausgebildet. Wie diese Windelhöschen hergestellt werden kann, ist der Druckschrift aber nicht zu entnehmen.

US-A-5,213,645 zeigt ein Verfahren zum Herstellen von Windel- oder Inkontinenzhöschen, wobei die herzustellenden Höschen durchgehend in ihrer späteren Querrichtung gefördert werden. Es werden in Förderrichtung verlaufende, die spätere Hüftöffnung elastifizierende Mittel durchgehend eingebracht. Danach wird die Bahn um eine in Querrichtung der herzustellenden Höschen und damit in Förderrichtung verlaufende Achse gefaltet. Nach Ausstanzen von die Beinöffnungen bildenden Öffnungen wird die Bahn quer zur Förderrichtung geschnitten, und es werden Siegellinien angebracht, welche die gefalteten Abschnitte verbinden und die beidseitigen Seitennähte der Höschen bilden. Eine Drehung und Vereinzelung der Höschen während der Herstellung findet nicht statt. Es ist nicht möglich, einen in Querrichtung über einen Hauptteil vorstehenden Hüftgurtabschnitt zu bilden.

Hiervon ausgehend liegt der vorliegenden Erfindung die Aufgabe zugrunde, ein kostengünstiges und störungsunanfälliges Verfahren zum Herstellen eines Windeloder Inkontinenzhöschen der eingangs genannten Art zu schaffen.

Diese Aufgabe wird durch ein Verfahren mit den Merkmalen der nebengeordneten Ansprüche 1 und 5 gelöst.

Es wäre beim Herstellungsverfahren nach Anspruch 1 ansich denkbar, dass die den Hüftabschnitt bildenden Flachmaterialbahnen bevor sie miteinander unlösbar verbunden werden, quer zu ihrer Zuführrichtung in Teilabschnitte getrennt werden. Dies erweist sich jedoch angesichts der Vorspannung, unter welcher die zugeführten Flachmaterialbahnen stehen, als nachteilig.

Bevorzugtermaßen wird eine derart breite Verbindungsnaht, etwa in Form einer Siegelnaht oder eines Kleberauftrags vorgesehen, dass die Trennung der Bahnen anschließend durch einen quer zur Zuführrichtung und durch den Verbindungsbereich laufenden Trennschnitt ausgeführt werden kann, ohne dass die unlösbare Verbindung der beiden Flachmaterialbahnen beeinträchtigt wird.

Es hat sich bei allen Verfahrensvarianten als zweckmäßig erwiesen, wenn die Flachmaterialbahnen am Vorderbereich und Rückbereich des gefalteten Teils nicht vollflächig sondern bereichsweise, z.B. punktuell oder in Form voneinander beabstandeter Streifen/Linien oder in Form eines ein zusammenhängendes Netzwerk bildenden Musters, fixiert werden. Dies hat den Vorteil, dass die Verbindung zwischen den vorgespannten Flachmaterialbahnen und dem Vorder- und Rückbereich weniger steif ist, so dass das Höschen im fertigen Zustand auch im Vorder- und Rückbereich des Produktes leicht zusammenziehbar und dehnbar ist. Zudem kann durch die Geometrie der fixierten Bereiche eine definierte, gefällig wirkenden Rüschung des Vorder- und Rückbereiches erzielt werden.

Es erweist sich als vorteilhaft, wenn die Flachmaterialbahnen so zugeführt werden, dass sie den Vorderbereich und Rückbereich des gefalteten Teils über dessen Längsenden von Vorderbereich und Rückbereich überlappen und dementsprechend nur über einen Teil ihrer Breite mit dem Vorderbereich und Rückbereich unlösbar verbunden werden.

Der insbesondere sanduhrförmige Hauptteil des Höschens, kann sehr schmal bauend ausgebildet werden. Im einfachsten Fall kann es sich um eine rechteckförmige Vorlage handeln, bei welcher Vorderbereich und Rückbereich in Längsrichtung nicht breiter "bauen" als der im Schrittbereich eines Benutzers zu liegen kommende Mittelbereich.

Der gürtelförmige Hüftabschnitt kann durch auf dem einschlägigen Gebiet übliche Fügeverfahren, insbesondere Siegeln oder Verbinden mittels Schmelzkleber, am Vorderbereich und am Rückbereich des insbesondere sanduhrförmigen Teils unlösbar befestigt werden.

Die elastischen Materialabschnitte werden nicht voneinander isoliert an den insbesondere sanduhrförmigen Hauptteil, das "Chassis", angestückt, sondern sie bilden einen in Umfangsrichtung geschlossenen Hüftabschnitt, der in Umfangsrichtung durchgehend elastisch ausgebildet ist.

Es hat sich als besonders vorteilhaft erwiesen, wenn ein Längsende des Rückbereichs und des Vorderbereichs des Höschens einen Abstand zu einem Längsende des in Umfangsrichtung geschlossenen Hüftabschnitts aufweist, d.h. wenn das Längsende des "Chassis" nicht bündig mit dem Längsende des Hüftabschnitts verläuft. Hierdurch kann eine Materialersparnis erzielt werden, und in Folge der Querelastizität, d.h. Elastizität in Umfangsrichtung des gürtelförmigen Hüftabschnitts entsteht bei einer entsprechenden Höhe des Hüftabschnitts von einigen Zentimetern ein sehr gefällig wirkendes, optisches Erscheinungsbild des Bundbereichs des Höschens. Im Übrigen werden Produktionsungenauigkeiten verdeckt oder fallen nicht auf, da der Hüftabschnitt in Folge der Elastizität zum Zusammenziehen neigt.

Der gürtelförmig in Umfangsrichtung geschlossene Hüftabschnitt kann aus einem im Wesentlichen in einer Richtung elastischen Material gebildet sein. Dies vermittelt ein angenehmeres Traggefühl als ein in jeder Richtung seiner flächenhaften Erstreckung elastisches Material.

In bevorzugter Weise ist der gürtelförmige in Umfangsrichtung geschlossene Hüftabschnitt von zwei Vliesschichten und dazwischen angeordneten in einer Richtung erstreckten Elastifizierungsmitteln, insbesondere gummielastischen Fäden, gebildet. Hierdurch wird einerseits der als positiv empfundene Anblick einer textilen Oberfläche vermittelt, und andererseits ein angenehmes Traggefühl vermittelt.

Bei einer ganz besonders bevorzugten Ausführungsform des Höschens wird das Elastifizierungsmittel bereits im vorgespannten Zustand mit den beiden Vliesschichten verbunden.

Nach der zweiten Ausführungsform der Erfindung (Anspruch 5) wird aus einer ersten und einer zweiten Flachmaterialbahn, die aus einem Mittelteil und in Längsrichtung seitlich angestückten elastischen Bereichen oder aber aus einem durchgehend elastischen Material gebildet sind, eine geschlossene schlauchförmige Bahn hergestellt. Diese schlauchförmige Bahn wird dann zum Bilden des in Umfangsrichtung geschlossenen gürtelförmigen Hüftabschnitts in Querrichtung, also senkrecht zur Längsrichtung, geschnitten. Der gürtelförmige Hüftabschnitt wird also beim Zuführen der ersten und zweiten Flachmaterialbahn bzw. des Schlauchs in Längsrichtung als quer zur Längsrichtung abgetrennter Längsabschnitt hergestellt. Dieser Längsabschnitt wird dann mit dem Vorderbereich und dem Rückbereich des ebenfalls in Längsrichtung geförderten insbesondere sanduhrförmigen Hauptteils des herzustellenden Höschens unlösbar fixiert. Der in Längsrichtung geförderte Hauptteil kann auch vor dem Fixieren um eine quer zur Längsrichtung verlaufende Achse derart gefaltet werden, dass Vorderbereich bzw. Rückbereich übereinander zu liegen kommen.

Bei der zweiten Ausführungsform der Erfindung erweist es sich als vorteilhaft, dass die Förderrichtung der Hauptteile beibehalten werden kann.
- Die Figuren 1a bis c: zeigen schematisch den erfindungsgemäßen Herstellungsablauf eines Windel- oder Inkontinenzhöschens und
- Figur 2: zeigt schematisch den Aufbau des bevorzugtermaßen zur Ausbildung des gürtelförmigen Hüftabschnitts verwendbaren Materials und
- Figuren 3a bis 3e: eine schematische Darstellung des Herstellungsverfahrens nach der zweiten Ausführungsform der Erfindung.

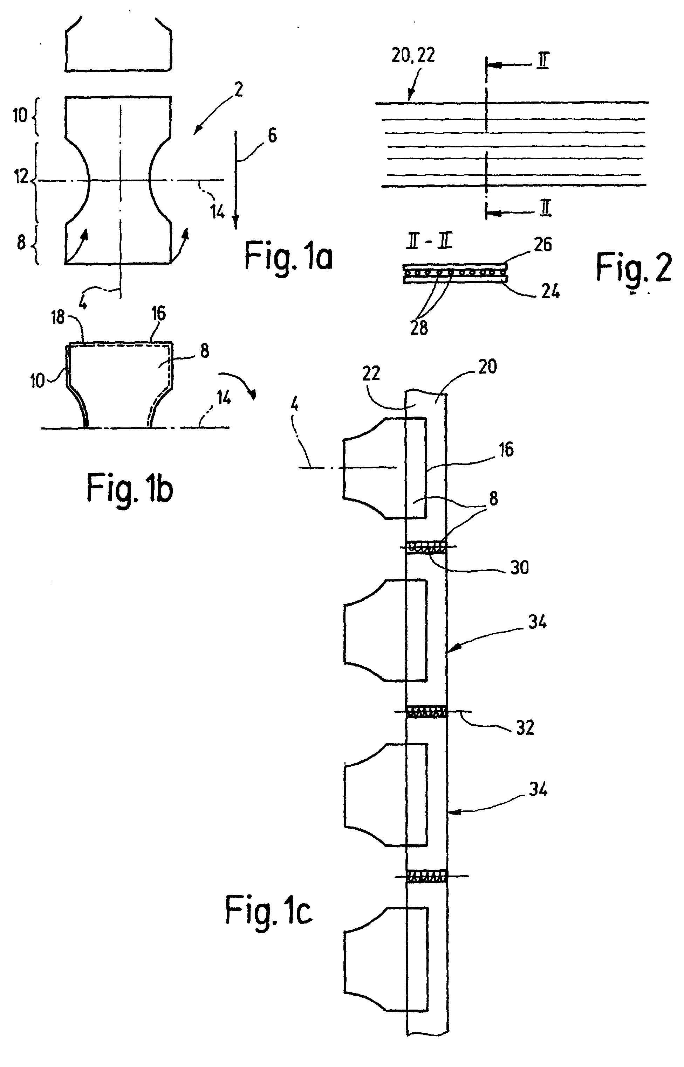

Wie in Figur 1a schematisch dargestellt, wird ein zuvor in ansich bekannter und daher an dieser Stelle nicht zu beschreibender Weise hergestellter sanduhrförmiger Hauptteil 2 in seiner Längsrichtung 4 in Produktionsrichtung 6 einer schnelllaufenden Maschine gefördert. Der sanduhrförmige Hauptteil 2 umfasst einen Vorderbereich 8, einen Rückbereich 10 und einen dazwischen liegenden Mittelbereich 12, der bogenförmige Ausnehmungen für die Beine des Trägers des Höschens aufweist. Der sanduhrförmige Hauptteil 2 kann in an sich üblicher Weise eine die körperabgewandte Außenseite bildende flüssigkeitsdichte Schicht, einen vorzugsweise SAP-Materialien umfassenden Saugkörper und eine die körperzugewandte Innenseite bildende, zumindest im Bereich des darunter angeordneten Saugkörpers flüssigkeitsdurchlässige Schicht aufweisen.

In einem weiteren Herstellungsschritt wird der sanduhrförmige Hauptteil um eine Symmetrieachse 14 derart gefaltet, dass Vorderbereich 8 und Rückbereich 10 mit bündig zueinander verlaufenden Längsenden 16, 18 übereinander zu liegen kommen.

Der so gefaltete Hauptteil wird um eine senkrecht zur Zeichnungsebene der Figur 1b verlaufende Achse gedreht, so dass die gefalteten Hauptteile - wie in Figur 1c angedeutet - quer zu ihrer Längsrichtung 4, d.h. in Richtung der Längsenden 16, 18 gefördert werden.

Von oberhalb und unterhalb des gefalteten Hauptteils 2 wird je eine Flachmaterialbahn 20, 22 zugeführt, die in Zuführrichtung elastisch ausgebildet ist und in vorgedehntem oder vorgespanntem Zustand zugeführt wird. Die Flachmaterialbahnen 20, 22 bestehen jeweils aus zwei in Figur 2 dargestellten Vliessschichten 24, 26 zwischen denen elastische Fäden 28 in vorgespanntem Zustand vorgesehen wurden.

Die Bahnen 20, 22 werden so zugeführt, dass sie die jeweilige Außenseite von Vorderbereich 8 und Rückbereich 10 etwa mit der Hälfte ihrer Breite überlappen. Die Bahnen 20, 22 werden durch bereichsweisen Kleberauftrag an der Außenseite des Vorderbereichs 8 und Rückbereichs 10 fixiert.

Die Bahnen 20, 22 werden - immer noch unter Vorspannung stehend - im Bereich zwischen zwei gefalteten Hauptteilen unlösbar miteinander verbunden und zwar entlang einer quer zur Zuführrichtung verlaufenden Linie 30. Der Verbindungsbereich ist dabei zumindest so breit, dass unmittelbar danach oder an einer in Produktionsrichtung nachfolgenden Stelle ein quer zur Zuführrichtung der Flachmaterialbahn 20, 22 verlaufender Trennschnitt, der mit dem Bezugszeichen 32 angedeutet ist, ausgeführt werden kann, ohne dass die unlösbare Verbindung der Bahnen gelöst wird. Hierdurch wird ein in Umfangsrichtung geschlossener Hüftabschnitt 34 durch die Flachmaterialbahnen 20, 22 gebildet. Nach dem Ausführen des Trennschnitts sind die Windel- oder Inkontinenzhöschen fertiggestellt und werden einer Verpackungsstation zugeführt.

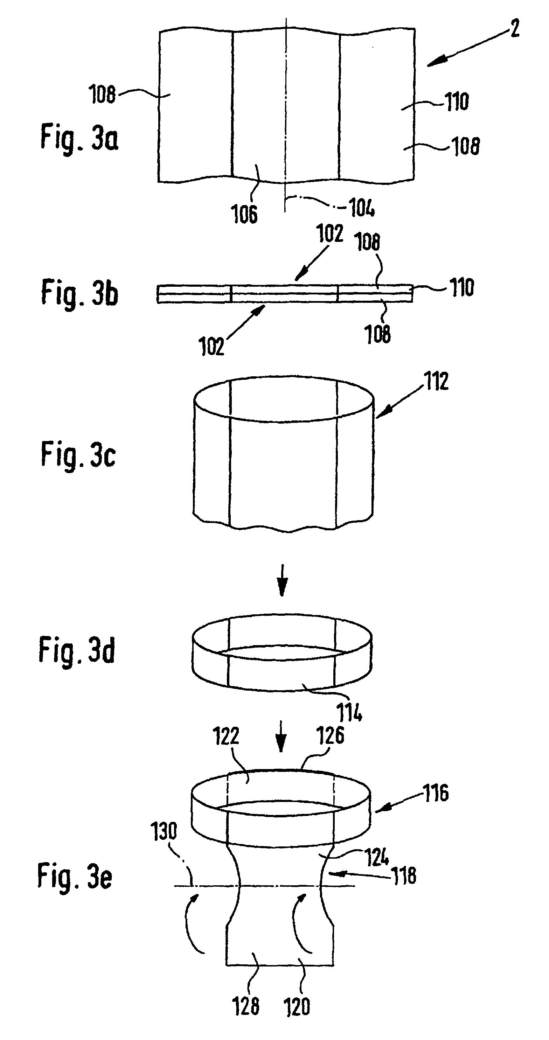

Die Figuren 3a bis 3e zeigen schematisch ein Herstellungsverfahren nach der zweiten Ausführungsform der Erfindung für ein Windel- oder Inkontinenzhöschen. Es werden vorzugsweise zwei in Figur 3a mit dem Bezugszeichen 102 angedeutete Flachmaterialbahnen bereitgestellt, die einen in Längsrichtung 104 erstreckten im wesentlichen unelastischen Mittelteil 106 und seitlich angestückte ebenfalls in Längsrichtung 104 erstreckte und quer bzw. senkrecht zur Längsrichtung 104 elastische Materialabschnitte 108 aufweisen. Die zwei Flachmaterialbahnen 102 werden dann, wie in Figur 3b angedeutet, übereinander positioniert und im Bereich ihrer seitlich äußeren Längsrandabschnitte 110 der querelastischen Materialabschnitte 108 miteinander verbunden, so dass eine in Figur 3c angedeutete schlauchförmige Bahn 112 gebildet ist. Von dieser schlauchförmigen Bahn 112 werden Längsabschnitte 114 quer zur Längsrichtung 104 abgetrennt, die dann einen gürtelförmigen Hüftabschnitt 116 des herzustellenden Windel- oder Inkontinenzhöschens bilden. Dieser Hüftabschnitt 116 wird an einem sanduhrförmigen Hauptteil 118 fixiert. Dieser sanduhrförmige Hauptteil 118 umfasst einen Vorderbereich 120, einen Rückbereich 122 und einen im Schrittbereich eines Benutzers des Höschens zu liegen kommenden Mittelbereich 124, der zur Bildung von Beinausschnitten sanduhrförmig verschlankt ausgebildet ist. Der gürtelförmige Hüftabschnitt 116 verläuft bündig mit dem Längsrand 126 des Rückbereichs 122 und mit dem Längsrand 128 des Vorderbereichs 120 des sanduhrförmigen Hauptteils 118. Zum Fixieren des Hüftabschnitts 116 wird der sanduhrförmige Hauptteil 118 um eine quer zur Längsrichtung 4 verlaufende Achse 130 gefaltet.

## Patentansprüche

1. Verfahren zum Herstellen von Windel- oder Inkontinenzhöschen zum einmaligen Gebrauch, mit einem Hauptteil (2), bestehend aus einem Vorderbereich (8), einem Rückbereich (10) und einem in Längsrichtung (4) dazwischen liegenden im Schrittbereich des Benutzers des Höschens zu liegen kommenden Mittelbereich (12), wobei der Hauptteil (2) eine die körperabgewandte Außenseite bildende, zumindest abschnittsweise flüssigkeitsdichte Schicht, einen Saugkörper und eine die körperzugewandte Innenseite bildende, zumindest im Bereich des darunter angeordneten Saugkörpers flüssigkeitsdurchlässige Schicht aufweist, und mit elastischen Materialabschnitten, welche den Vorderbereich (8) und den Rückbereich (10) zur Bildung der Höschenform miteinander verbinden und sich in senkrecht zur Längsrichtung (4) verlaufender Querrichtung über seitliche Längsränder des Hauptteils (2) hinaus erstrecken, wobei die elastischen Materialabschnitte einen gürtelförmigen in Umfangsrichtung geschlossenen Hüftabschnitt (34) bilden, der am Rückbereich (10) und am Vorderbereich (8) des Hauptteils (2) unlösbar befestigt ist, mit folgenden Verfahrensschritte in ihrer nachfolgend gegebenen Reihenfolge:
- Bilden eines jeweiligen Vorder-, Rück- und Mittelbereich (8, 10, 12) umfassenden Hauptteils (2) und Fördern des jeweiligen Hauptteils (2) in seiner Längsrichtung (4),
- Falten des jeweiligen Hauptteils (2) um eine senkrecht zur Längsrichtung (4) verlaufende Achse (14) derart, dass Vorderbereich (8) und Rückbereich (10) mit ihren Längsenden (16, 18) bündig übereinander zu liegen kommen,
- Drehen des jeweiligen gefalteten Hauptteils (2) um 90 Grad um eine zur Förderebene senkrechte Achse, so dass der jeweilige Hauptteil (2) quer zu seiner Längsrichtung (4) und in einem Abstand zu benachbarten Hauptteilen weitergefördert wird,
- Zuführen zweier den gürtelförmigen Hüftabschnitt (34) bildenden Flachmaterialbahnen (20, 22) in Förderrichtung der jeweiligen gefalteten Hauptteile (2), wobei die beiden Flachmaterialbahnen (20, 22) aus einem elastischen Material bestehen oder elastifiziert ausgebildet sind und im in Zuführrichtung vorgedehnten Zustand oberhalb bzw. unterhalb des jeweiligen gefalteten Hauptteils (2) zugeführt werden,
- Fixieren der einen Flachmaterialbahn (20) am Vorderbereich (8) und der anderen (22) am Rückbereich (10) des gefalteten Hauptteils (2) und unlösbares Verbinden der beiden Flachmaterialbahnen (20, 22) miteinander quer zu ihrer Zuführrichtung und zur Förderrichtung des gefalteten Hauptteils (2) und in einem Abstand zum Hauptteil (2),
- Schneiden der Flachmaterialbahnen (20, 22) quer zu ihrer Zuführrichtung und zur Förderrichtung des gefalteten Hauptteils (2) zum Bilden voneinander separierbarer Höschen.

2. Verfahren nach Anspruch 1, **dadurch gekennzeichnet, dass** die beiden Flachmaterialbahnen (20, 22) über einen in Zuführrichtung derart breiten Abschnitt unlösbar miteinander verbunden werden, dass im Bereich dieses Abschnitts ein Trennschnitt (32) quer zur Zuführrichtung ausgeführt werden kann, ohne die unlösbare Verbindung zu beeinträchtigen.

3. Verfahren nach Anspruch 1 oder 2, **dadurch gekennzeichnet, dass** die Flachmaterialbahnen (20, 22) bereichsweise am Vorderbereich (8) und Rückbereich (10) des gefalteten Hauptteils (2) fixiert werden.

4. Verfahren nach Anspruch 1, 2 oder 3, **dadurch gekennzeichnet, dass** die Flachmaterialbahnen (20, 22) so zugeführt werden, dass sie den Vorderbereich (8) und Rückbereich (10) des gefalteten Hauptteils (2) über dessen Längsenden (16, 18) hinweg überlappen.

5. Verfahren zum Herstellen von Windel- oder Inkontinenzhöschen zum einmaligen Gebrauch, mit einem Hauptteil (118), bestehend aus einem Vorderbereich (120), einem Rückbereich (122) und einem in Längsrichtung (104) dazwischen liegenden im Schrittbereich des Benutzers des Höschens zu liegen kommenden Mittelbereich (124), wobei der Hauptteil (118) eine die körperabgewandte Außenseite bildende, zumindest abschnittsweise flüssigkeitsdichte Schicht, einen Saugkörper und eine die körperzugewandte Innenseite bildende, zumindest im Bereich des darunter angeordneten Saugkörpers flüssigkeitsdurchlässige Schicht aufweist, und mit elastischen Materialabschnitten (108), welche den Vorderbereich (120) und den Rückbereich (122) zur Bildung der Höschenform miteinander verbinden und sich in senkrecht zur Längsrichtung (104) verlaufender Querrichtung über seitliche Längsränder des Hauptteils (118) hinaus erstrecken, wobei die elastischen Materialabschnitte (108) einen gürtelförmigen in Umfangsrichtung geschlossenen Hüftabschnitt (114) bilden, der am Rückbereich (122) und am Vorderbereich (120) des Hauptteils (118) unlösbar befestigt ist, mit folgenden Verfahrensschritten in der nachfolgend angegebenen Reihenfolge:
- Bilden von Vorder-, Rück- und Mittelbereich (120, 122, 124) umfassenden Hauptteilen (118) und Fördern der jeweiligen Hauptteile in ihrer Längsrichtung (104),
- Bilden einer ersten und einer zweiten Flachmaterialbahn (102), die aus einem mittleren Längsbereich (106) und in Längsrichtung (104) seitlich angestückten elastischen Bereichen (108) oder aus einem durchgehend elastischen Material gebildet sind, wobei die Breite der Flachmaterialbahn (102) größer ist als die Breite des Hauptteils in seiner Querrichtung und Fördern der Flachmaterialbahnen (102) übereinander in Längsrichtung (104) und Ablegen der Flachmaterialbahnen (102) übereinander,
- Fixieren der ersten und der zweiten übereinander abgelegten Flachmaterialbahnen (102) miteinander in seitlichen Längsrandbereichen (110) zum Bilden einer geschlossenen schlauchförmigen Bahn (112),
- Querschneiden der schlauchförmigen Bahn (112) zum Bilden eines jeweiligen in Umfangsrichtung geschlossenen gürtelförmigen Hüftabschnitts (116),
- Fixieren des jeweiligen gürtelförmigen Hüftabschnitts (116) am Vorderbereich (120) und am Rückbereich (122) eines jeweiligen Hauptteils (118).

## Claims

1. Method for producing disposable nappies or incontinence pants, with a main part (2) consisting of a front region (8), a back region (10) and a central region (12) located therebetween in the longitudinal direction (4) and coming to rest in the crotch region of the user of the pants, wherein the main part (2) has a layer which is liquid-tight at least in certain portions, and forms the body-remote outer side, an absorbent member and a layer which is liquid-permeable at least in the region of the absorbent member arranged therebelow and forms the inner side facing the body, and with elastic material portions connecting the front region (8) and the back region (10) to one another to form the pant shape and extending beyond lateral longitudinal edges of the main part (2) in the transverse direction running perpendicular to the longitudinal direction (4), the elastic material portions forming a belt-like hip portion (34) closed in the circumferential direction and non-detachably fastened to the back region (10) and the front region (8) of the main part (2), with the following method steps in their sequence given hereinafter:
- forming a main part (2) comprising a respective front, back and central region (8 10, 12) and conveying the respective main part (2) in its longitudinal direction (4),
- folding the respective main part (2) about an axis (14) extending perpendicular to the longitudinal direction (4) in such a way that the front region (8) and back region (10) come to rest with their longitudinal ends (16, 18) flush one above the other,
- rotating the respective folded main part (2) by 90° about an axis perpendicular to the conveying plane so the respective main part (2) is conveyed onwards transversely to its longitudinal direction (4) and at a distance from adjacent main parts,
- supplying two flat material webs (20, 22) forming the belt-like hip portion (34) in the conveying direction of the respective folded main parts (2), the two flat material webs (20, 22) consisting of an elastic material or being elasticated in design and being supplied in the prestretched state in the supply direction above or below the respective main part (2),
- fixing the one flat material web (20) to the front region (8) and the other (22) to the back region (10) of the folded main part (2) and non-detachable connection of the two flat material webs (20, 22) to one another transversely to their supply direction and to the conveying direction of the folded main part (2) and at a distance from the main part (2),
- cutting the flat material webs (20, 22) transversely to their supply direction and to the conveying direction of the folded main part (2) to form pants which can be separated from one another.

2. Method according to claim 1, **characterised in that** the two flat material webs (20, 22) are non-detachably connected to one another by a wide portion in the supply direction such that a separating cut (32) can be made in the region of this portion transversely to the supply direction without affecting the non-detachable connection.

3. Method according to claim 1 or 2, **characterised in that** the flat material webs (20, 22) are fixed, at least in certain regions, to the front region (8) and back region (10) of the folded main part (2).

4. Method according to claim 1, 2, or 3, **characterised in that** the flat material webs (20, 22) are supplied in such a way that they overlap the front region (8) and back region (10) of the folded main part (2) over the longitudinal ends (16, 18) thereof.

5. Method for producing disposable nappies or incontinence pants, with a main part (118) consisting of a front region (120), a back region (122) and a central region (124) located therebetween in the longitudinal direction (104) and coming to rest in the crotch region of the user of the pants, wherein the main part (118) has a layer which is liquid-tight, at least in certain portions, and forms the body-remote outer side, an absorbent member and a layer which is liquid-permeable at least in the region of the absorbent member arranged therebelow and forms the inner side facing the body, and with elastic material portions (108) connecting the front region (120) and the back region (122) to one another to form the pant shape and extending beyond lateral longitudinal edges of the main part (118) in the transverse direction running perpendicular to the longitudinal direction (104), the elastic material portions (108) forming a belt-like hip portion (34) closed in the circumferential direction and non-detachably fastened to the back region (122) and the front region (120) of the main part (118), with the following method steps in the sequence given hereinafter:
- forming main parts (118) comprising a front, back and central region (120, 122, 124) and conveying the respective main parts in their longitudinal direction (104),
- forming a first and a second flat material web (102) formed from a central longitudinal region (106) and elastic regions (108) attached laterally in the longitudinal direction (104) or from a continuously elastic material, wherein the width of the flat material web (102) is greater than the width of the main part in its transverse direction, and conveying the flat material webs (102) one above the other in the longitudinal direction (104), and stacking the flat material webs (102) one above the other,
- mutually fixing the first and the second flat material webs (102) stacked one above the other in lateral longitudinal edge regions (110) to form a closed tubular web (112),
- transversely cutting the tubular web (112) to form a respective belt-like hip portion (116) closed in the circumferential direction,
- fixing the respective belt-like hip portion (116) to the front region (120) and back region (112) of a respective main part (118).

## Revendications

1. Procédé pour la fabrication de culottes de couches ou de garnitures pour l'incontinence, à usage unique, comportant une partie principale (2) qui est constituée d'une zone avant (8), d'une zone arrière (10) et d'une zone médiane (12) qui vient se placer, dans la direction longitudinale (4), entre les deux autres zones, dans la zone de marche de l'utilisateur de la culotte, la partie principale (2) comportant une couche, qui est, au moins par sections, étanche aux liquides et qui forme la face extérieure dirigée vers le corps, un corps absorbant et une couche qui est perméable aux liquides au moins dans la zone du corps absorbant disposé en dessous, et des sections de matière élastique qui relient la zone avant (8) et la zone arrière (10) entre elles pour la réalisation d'une forme de culotte et qui s'étendent dans une direction transversale allant perpendiculairement à la direction longitudinale (4), au dessus de bords longitudinaux latéraux de la partie principale (2), les sections de matière élastique constituant une section de hanche (34) en forme de ceinture qui est fermée dans la direction périphérique et qui est fixée de manière indétachable sur la zone arrière (10) et sur la zone avant (8) de la partie principale (2), ledit procédé comportant les étapes de procédé suivantes selon la séquence indiquée ci-après :
- former une partie principale (2) comportant, à chaque fois, une zone avant (8), une zone arrière (10) et une zone médiane (12) et déplacer chaque partie principale (2) dans sa direction longitudinale (4),
- plier chaque partie principale (2) autour d'un axe (14) qui s'étend perpendiculairement à la direction longitudinale (4) de telle manière que la zone avant (8) et la zone arrière (10) viennent se placer l'une sur l'autre avec affleurement,
- faire tourner la partie principale (2) pliée de 90 degrés autour d'un axe perpendiculaire au plan de déplacement de telle manière que la partie principale (2) soit déplacée de nouveau dans une direction transversale par rapport à sa direction longitudinale (4) et à une certaine distance des parties principales voisines,
- amener deux bandes de matière plates (20, 22), qui forment la section de hanche (34) en forme de ceinture dans la direction de déplacement de chacune des parties principales (2) pliées, les deux bandes de matière plates (20, 22) étant constituées de matière élastique ou étant conformées pour être rendues élastiques et étant amenées dans un état de tension dans la direction d'amenée préalable au dessus ou au dessous de chaque partie principale (2) pliée,
- fixer l'une des bandes de matière plates (20) sur la zone avant (8) et l'autre (22) sur la zone arrière (10) de la partie principale (2) pliée et relier de manière indétachable les deux bandes de matière plates (20, 22) entre elles selon une direction transversale par rapport à leur direction d'amenée et à la direction de déplacement de la partie principale (2) pliée et à une certaine distance de la partie principale (2),
- découper les bandes de matière plates (20, 22) selon une direction transversale par rapport à leur direction d'amenée et à la direction de déplacement de la partie principale (2) pliée pour former des culottes pouvant être séparées l'une de l'autre.

2. Procédé selon la revendication 1, **caractérisé en ce que** les deux bandes de matière plates (20, 22) sont reliées entre elles de manière indétachable par l'intermédiaire d'une section dont la largeur selon la direction d'amenée est telle que, dans la zone de cette section, on peut réaliser une couche de séparation (32) selon une direction transversale par rapport à la direction d'amenée, sans affecter la liaison indétachable.

3. Procédé selon la revendication 1 ou 2, **caractérisé en ce que** les bandes de matière plates (20, 22) sont fixées par zones sur la zone avant (8) et la zone arrière (10) de la partie principale (2) pliée.

4. Procédé selon l'une quelconque des revendications 1, 2 ou 3, **caractérisé en ce que** les bandes de matière plates (20, 22) sont amenées de telle manière qu'elles sont en recouvrement avec la zone avant (8) et la zone arrière (10) de la partie principale (2) pliée au dessus des extrémités longitudinales (16, 18) de cette dernière.

5. Procédé pour la fabrication de culottes de couches ou de garnitures pour l'incontinence, à usage unique, comportant une partie principale (118) qui est constituée d'une zone avant (120), d'une zone arrière (122) et d'une zone médiane (124) qui vient se placer, dans la direction longitudinale (104), entre les deux autres zones, dans la zone de marche de l'utilisateur de la culotte, la partie principale (118) comportant une couche, qui est, au moins par sections, étanche aux liquides et qui forme la face extérieure dirigée vers le corps, un corps absorbant et une couche qui est perméable aux liquides au moins dans la zone du corps absorbant disposé en dessous, et des sections de matière élastique (108) qui relient la zone avant (120) et la zone arrière (122) entre elles pour la réalisation d'une forme de culotte et qui s'étendent dans une direction transversale allant perpendiculairement à la direction longitudinale (104), par l'intermédiaire de bords longitudinaux latéraux de la partie principale (118), les sections de matière élastique (108) constituant une section de hanche (114) en forme de ceinture qui est fermée dans la direction périphérique et qui est fixée de manière indétachable sur la zone arrière (122) et sur la zone avant (120) de la partie principale (120), ledit procédé comportant les étapes de procédé suivantes selon la séquence indiquée ci-après :
- former des parties principales (118) comportant une zone avant (120), une zone arrière (122) et une zone médiane (124) et déplacer chacune des parties principales (2) selon sa direction longitudinale (104),
- former une première et une deuxième bandes de matière plates (102) qui sont constituées d'une zone longitudinale médiane (106) et de zones élastiques (108) raboutées latéralement dans la direction longitudinale (104) ou d'une matière élastique de manière continue, la largeur de la bande matière plate (102) étant plus grande que la largeur de la partie principale dans sa direction, transversale, et déplacer les bandes de matière plates (102) l'une sur l'autre dans la direction longitudinale (104) et déposer les bandes de matière plates (102) l'une sur l'autre,
- fixer entre elles les première et deuxième bandes de matière plates (102), qui sont déposées l'une sur l'autre, dans des zones de bord longitudinales latérales (110) pour former une bande fermée en forme de tuyau (112),
- couper la bande en forme de tuyau (112) selon la direction transversale pour former, à chaque fois, une section de hanche (116) en forme de ceinture qui est fermée dans la direction périphérique,
- fixer chacune des sections de hanche (116) en forme de ceinture sur la zone avant (120) et la zone arrière (122) de chaque partie principale (118).
